(19) Europäisches Patentamt
European Patent Office
Office européen des brevets

(11) **EP 4 603 060 A1**

(12) **EUROPEAN PATENT APPLICATION**

(43) Date of publication:
**20.08.2025 Bulletin 2025/34**

(21) Application number: **24157743.6**

(22) Date of filing: **15.02.2024**

(51) International Patent Classification (IPC):
*A61F 2/24* *(2006.01)*

(52) Cooperative Patent Classification (CPC):
**A61F 2/2403; A61F 2/2412**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Universität Bern**
**3012 Bern (CH)**

(72) Inventors:
• ZHENG, Shokai
  **3010 Bern (CH)**
• OBRIST, Dominik
  **3010 Bern (CH)**

(74) Representative: **Specht, Peter et al**
**Loesenbeck - Specht - Dantz
Patent- und Rechtsanwälte
Am Zwinger 2
33602 Bielefeld (DE)**

(54) **HEART VALVE**

(57)     A heart valve (1, 11), especially prosthetic heart valve, comprising an annular base body (5, 17) and at least one leaflet (2, 3, 13, 14, 15) movably arranged relative to the base body (5, 17), wherein the heart valve (1, 11) has an opening state and a closed state, wherein the leaflet (2, 3, 13, 14, 15) has at least a serrated edge profile (6, 7, 16).

# Fig. 2

EP 4 603 060 A1

## Description

### Technical Field

The current invention relates to a prosthetic heart valve with a new athrombogenic design.

Currently, more than 70 million people are affected by valvular heart diseases worldwide and more than 1 million deaths are reported each year. For patients requiring heart valve replacement, current guidelines in the EU and US recommend mechanical heart valve prosthesis for patients under 60 and 50 years old, respectively, in the aortic position, and under 65 years old in the mitral position. Yet, due to its significant impact on blood, often characterized by unphysiological shear stress level, mechanical heart valve often leads to a higher risk of thrombosis and thromboembolic events, making patients burdened with life-long anticoagulation such as Warfarin. Nonadherence may lead to an increased risk of bleeding and death. Consequently, the quest has never ceased for a mechanical heart valve with physiological flow characteristics identified by lower pressure gradient, lower turbulent kinetic energy, and lower shear stress level.

The current gold standard mechanical heart valve is the On-X valve distributed by Artivion, which is the only mechanical heart valve approved by FDA with less anticoagulation requirements. The design of On-X valve relied heavily on hydrodynamic principles, such as the flared entrance to alleviate flow separation, the large length to diameter ratio of 0.62 that reduces pressure gradient, and the large leaflet opening angle to minimize the wake. Subsequent studies have corroborated the reduced pressure gradient, turbulent kinetic energy and shear stress level in the wake and demonstrated good clinical outcome with lower anticoagulation level.

To go beyond, two strategies have been actively developed to create more physiological flow in mechanical heart valve. One is the implementation of passive flow control methods in existing bi-leaflet mechanical heart valve, and the other is the ongoing development of the tri-leaflet mechanical heart valve.

The concept of passive flow control method comprises the inclusion of vortex generators on mechanical heart valve leaflets as small rectangular structures on the surface of the leaflets, organized in co-rotating or counter-rotating patterns. From simulations and two-dimensional Particle Image Velocimetry measurements, the authors argued that co-rotating vortex generator is the best performing design in reducing pressure gradient and shear stress level by reducing the local flow separation on the leaflets. A later work confirmed these findings using direct numerical simulation, arguing that the vortex generators provide instabilities that corrupt the Kármán-like vortex shedding downstream of the leaflets, thus reducing the intensity of turbulent kinetic energy and lowering the shear stress level. In another recent direct numerical simulation study by Zolfaghari et al., by optimizing the shape of the leading edge, the authors succeeded in suppressing the turbulent wake and, at the same time, revealed the sensitivity of the mechanical heart valve-generated turbulence to the geometry of the leaflet's leading edge.

Another strategy is the relatively new tri-leaflet design that mimics the native valve morphology, such as the Triflo valve currently developed by Novostia SA, and the trileaflet design developed by Hans-Hinrich Sievers. Recent studies demonstrated that both effective orifice area and pressure gradient of the tri-leaflet design were improved against bi-leaflet mechanical heart valve, including On-X valve, and the shear stress level produced by the tri-leaflet mechanical heart valve was lower. The mechanism might be explained by the reduced intensity of shear layers in the wake, such that the intensity of the peripheral jets is much less than that in bi-leaflet mechanical heart valve and the mean velocity gradient is reduced.

### Background Art

As explained before, several design concepts for heart valves are known in the state of the art.

A developed design for heart valves is shown in US8834911B, which discloses a vortex generator with co-rotating or counter rotating layouts. It is disclosed that the pressure recovery was faster, and less turbulence was observed in the wake region.

Trileaflet valves belong to another class of mechanical valve design, that has shown potential in athrombogenic performance. Different patents, such as US9775708B2, US10182907B2, US20210212814A1, US2021021815A1, US20210212821A1, US10478288B2 and US20040249452A1 describe the general concept of this type of heart valve. Their design resembles the native aortic valve with three leaflets and mentions such designs produce more physiological flows.

### Object of the invention

It is an objective of the current invention to provide a new design for different heart valves with further improved athombogenic performance, which can preferably be applied to both mechanical and biological devices in the aortic, mitral and pulmonary positions of the heart

### Disclosure of the invention

The object is solved with a heart valve comprising the features of claim 1.

An inventive heart valve, especially a prosthetic heart valve, is adapted for a flow optimized design for a blood flow.

The inventive heart valve comprises an annular base body and at least one leaflet movably arranged relative to the base body. The heart valve has an opening state and a closed state.

According to the invention the leaflet has at least a serrated edge profile. Said edge profile may extend only partly or over the whole length of the edge of the leaflet, which is preferably arc-shaped.

Said serrated edge profile provides an optimized flow profile for a blood stream with under identical flow profile compared to a non-serrated edge profile.

Further embodiments are subject matter of the dependent claims.

According to a preferred embodiment the serrated edge profile comprises a wave contour with a sequence of a plurality of wave crests and wave troughs. Wave forms provide less turbulences than other serrated forms, such as a toothing or the like.

More preferably the wave contour is formed by combinations of sinusoidal waves which are preferably running in the same plane of the leaflet and which are more preferably formed as linear combinations of sinusoidal waves with varying amplitudes. The form and period of the waves provide further flow optimizations.

Said heart valve can be designed as a surgical heart valve, for example as a mechanical heart valve. The leaflet might preferably be arranged pivotably about a pivot axis and wherein the base body has an inner wall corresponding to the edge contour. This provides a preferred tight closure to the general constructional concept of the heart valve.

All leaflets of the heart valve may preferably have the serrated edge section, wherein the heart valve is preferably designed as a mono-leaflet, bi-leaflet or tri-leaflet heart valve for a further optimized flow.

It is preferred and of advantage if serrated edge profile is part of a downstream trailing side of the leaflet of the heart valve in the opening state and wherein the serrated edge profile is preferably part an arcuate shaped edge.

Even better flow conditions can be achieved if the heart valve has a second edge which is upstream front side of the leaflet of the heart valve in the opening state, wherein said second edge has a serrated, in particular wavy, and/or chamfered edge profile.

Said edge is positioned in the flow shadow of the leaflet and has less influence to the blood flow than the aforementioned arcuate shaped edge. The serration of said second edge can however be less advantageous for the tight closure of the heart valve.

The edge section of either the first edge and/or the second edge is at least partially straight or tapered, preferably tapered with a rounded or chamfered progress to one end section, wherein said end section more preferably protrudes over the whole length of said edge profile. This is a good compromise between the optimization of the flow profile and a tight closure of said heart valve.

In a preferred embodiment of the invention the wall has ribs forming a part of the said inner wall surface, which preferably extends linearly and also preferably have an increasing thickness towards the center along the longitudinal axis of the ring-shaped base body.

In a second alternative embodiment the wall may have ribs forming a part of the said inner wall surface which extend in an arcuate or spiral shape, preferably at an angle ($\alpha$) of between 0° and 45°, more preferably 1 and 40°, to the longitudinal axis of the annular base body. This assists the rotation of the blood flow as it comes out of the left ventricle of the heart.

The wall can have preferably arbitrary integer numbers larger than two of said ribs extending positioned with the same or varying distances from one another that is symmetric to the median plan of the leaflet which is of advantage for an enhanced optimization of the blood flow.

For a more body-adapted design the inventive heart valve can be provided as a biological heart valve, such as a surgical tissue valve, preferably with three leaflets.

An enhanced compatibility can be achieved if the heart valve is provided as a bioprosthetic heart valve, wherein the leaflet or leaflets are formed from organic material, in particular formed from organic tissue material.

To assist suturing, at least the cylindrical base body of a tissue valve can be made of inorganic material.

In a preferred embodiment at least the annular base body is made of organic or inorganic material and/or fabrics. In a more preferred embodiment at least the annular base body and/or the leaflets are made of titanium and/or titanium alloy. Even more preferably said titanium or titanium alloy can be partly or preferably fully coated with a carbon coating.

In a further preferred embodiment, the downstream trailing edges can preferably be designed according to equation

$$\begin{cases} x_t = -r * cos(t) \\ y_t = -r * sin(t) + a * cos(b * t) \end{cases}$$

for $-\pi \leq t \leq 0$, where $x_t$ and $y_t$ are spatial coordinates describing the shape of the edge, r is the radius of the valve leaflet, *sin* and *cos* are sinusoidal functions, a is proportional to the amplitude of the wave, and b is proportional to the number of crests of the wave.

**Brief Description of the drawings**

Some advantageous embodiments for an inventive heart valve are further explained in detail below together with drawings. Specific parts of the different embodiments can be understood as separate features that can also be realized in other embodiments of the invention. The combination of features described by the embodiment shall not be understood as a limitation for the invention.

Fig. 1     Perspective view of a first embodiment of a prosthetic heart valve which is not part of the invention in an opening state;

Fig. 2     Perspective view of a prosthetic heart valve as a first inventive embodiment in an opening state;

Fig. 3     Perspective view of a second embodiment of a prosthetic heart valve which is not part of the invention;

Fig. 4     Perspective view of a prosthetic heart valve as a second inventive embodiment;

Fig. 5     Perspective view of a first leaflet for the implementation in the first inventive embodiment;

Fig. 6     Perspective view of a second leaflet for the implementation in the first inventive embodiment;

Fig. 7     Perspective view of a third leaflet for the implementation in the first inventive embodiment;

Fig. 8     Perspective view of a fourth leaflet for the implementation in the first inventive embodiment;

Fig. 9     Perspective view of a first edge profile for the implementation in a leaflet according to one of the Fig. 5-8;

Fig. 10    Perspective view of a second edge profile for the implementation in a leaflet according to one of the Fig. 5-8;

Fig. 11    Perspective view of a third edge profile for the implementation in a leaflet according to one of the Fig. 5-8;

Fig. 12    Perspective view of an annular base body of the heart valve of Fig. 2;

Fig. 13    Top view of the annular base body of Fig. 12 perpendicular to the longitudinal axis L of the annular base body;

Fig. 14    Longitudinal cut view through the diameter D of the annular base body of Fig. 12 and 13;

Fig. 15    First perspective view of an annular base body of a further inventive embodiment of a heart valve;

Fig. 16    Second perspective view of the annular body of Fig. 15;

Fig. 17    Top view of the annular body of Fig. 15 and 16 perpendicular to the longitudinal axis L of the annular base body;

Fig. 18    Longitudinal cut view along the axis L of the annular body of Fig. 15, 16 and 17;

Fig. 19    Perspective view of the heart valve of Fig. 2 with the leaflets of Fig. 8 in a partly opening state;

Fig. 20    Perspective view of the heart valve of Fig. 19;

Fig. 21    Model of the aorta with aortic sinuses and with a heart valve in an up-stream position

Fig. 22    Isosurfaces of turbulent kinetic energy downstream of the mechanical heart valve of Fig. 1

Fig. 23    Isosurfaces of turbulent kinetic energy downstream of the inventive heart valve of Fig. 2.

**Mode for Carrying out the invention**

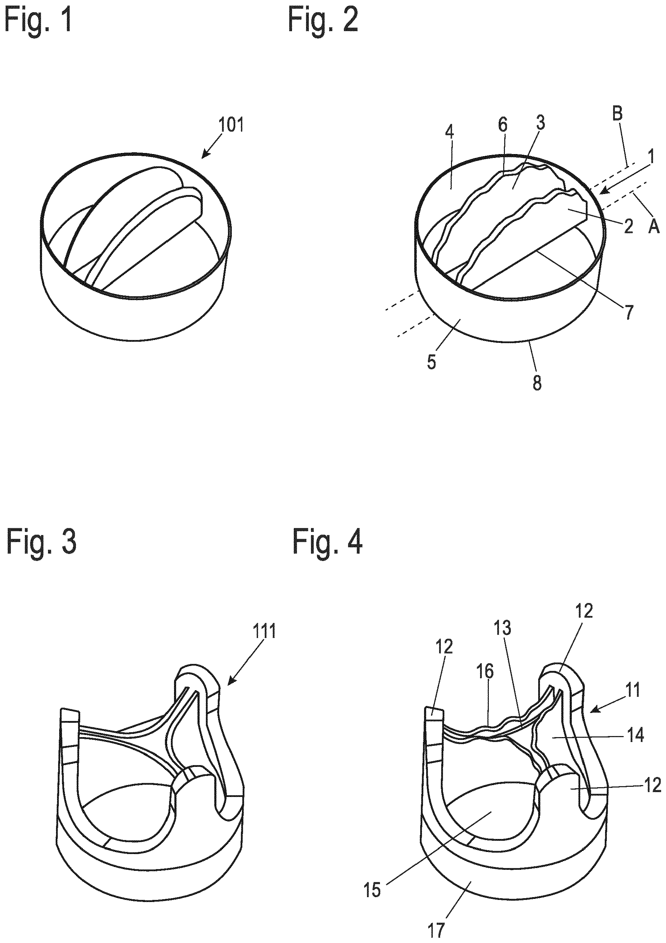

Fig. 1 and 3 discloses heart valves 101 and 111, which are not part of the invention being a bi-leaflet mechanical heart valve 101 and a tri-leaflet biological heart valve 111. Both leaflets have smooth edges in an arcuated shaped form.

Fig. 2 and 4 as well as Fig. 5-11, 19 and 20 show different designs of leaflets 2, 3, 13-15 for the implementation in inventive embodiments of heart valves 1 and 111.

Said heart valves are prosthetic heart valves. Fig. 2 shows a bi-leaflet mechanical heart valve and Fig. 4 shows a tri-leaflet biological heart valve. The heart valve of Fig. 2 is be provided as a mechanical heart valve whereas the embodiment of Fig. 4 can be provided either as a mechanical heart valve or as a bioprosthetic heart valve.

The heart valve 1 of Fig. 2 comprises an annular base body 5, herein also referred to as ring, and two leaflets 2 and 3.

Said annular base body 5 defines a longitudinal axis L and is provided with or formed a wall 8, with an inner surface, herein also referred to as inner wall surface 4, which correspond to an edge profile 6 of said leaflets 2 and 3. Said inner surface 4 is partly formed by longitudinal ribs and grooves positioned circumferentially with the same or varying distance to one each other. Said edge profile is part of a downstream trailing side where the blood stream leaves the leaflet, when the leaflet is in an opening state, such as shown in Fig. 2.

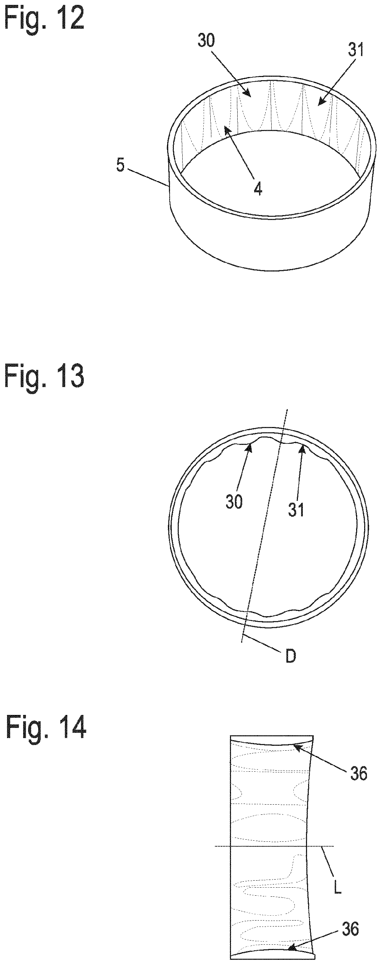

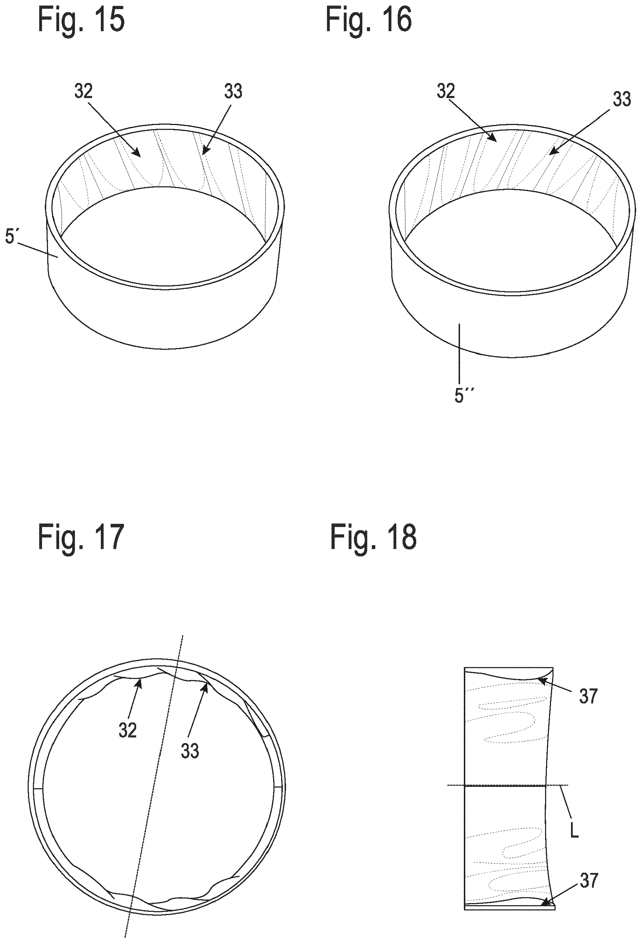

Said longitudinal ribs and grooves extend along the axis either as straight ribs 30 and grooves 31 or as arcuated and/or tilted ribs 32 and grooves 33. They will be further explained in the context of Fig. 12-14 and 15-18.

At both leaflet 2 and 3 in Fig. 2 are movably, preferably pivotably, arranged relative to the base body 5. They define each a pivot axis A and B positioned parallel to each other.

Said leaflets 2, 3 are further provided with a second edge 7 positioned upstream to the leaflet, with a front side contacting first an incoming blood stream.

In Fig. 2 the leaflets 2 and 3 are shaped as planar plates. Alternatively said leaflets can be shaped as corrugated plates with longitudinal corrugations that are parallel to said longitudinal axis L in an opening state of the heart valve 1. In both cases each leaflets define a plate plane which is parallel to said longitudinal axis L in an opening state and which can be angled arbitrarily between 40 to 90 degrees to said longitudinal axis L in a closed state.

The heart valve 1 of Fig. 2 can be changed from said opening state in said closed state by pivoting said leaflets 2, 3 about the pivot axis A and B.

The mechanical valve or at least one of the leaflets and/or the annular base body can be provided with titanium or with an titanium alloy. More preferably the titanium or titanium alloy body is partly or fully covered with a coating, more preferably with a carbon coating to reduce or prevent wearing and/or tearing.

Fig. 4 discloses a tri-leaflet heart valve 11 with three leaflets 13, 14, 15. The heart valve is provided with an annular base body 17 with three prongs 12. Said leaflets 13, 14, 15 are positioned between the prongs 12 and connect two of said prongs 12. In a closed state each of the three leaflets 13-15 are in direct contact with the other two leaflets 13-15.

The leaflets 13-15 are each provided with an edge profile 16 which is serrated, preferably with a wave contour, preferably defined by a combination of sinusoidal waves with varying amplitude. In a closed state said leaflets 13-15 are provided in a more concave form than in an opening state. The annular base body 17 can be provided with titanium or with a titanium alloy or with fabric material.

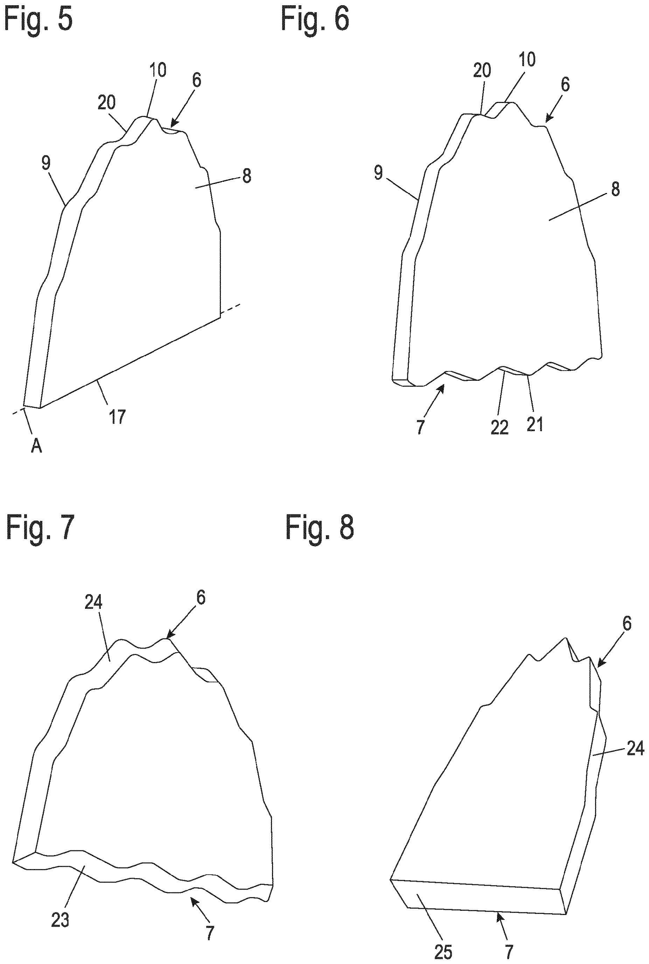

The leaflets 13-15 can be provided with a tissue, preferably an organic tissue. Said leaflets are provided with two main surfaces 8 and 9. The leaflets in Fig. 5-8 are provided with two edges 6, 7 wherein one edge 6 is arcuate shaped, herein also referred to as arc-shaped. The second edge 7 has a linear extension but could be provided with a serrated edge profile.

Said serrated edge profile of the edge 6 and/or of the edge 7 comprises wave crests 10 and wave troughs 20. The difference between Fig. 5-8 is implied in the different forms of the edge 7 at the upstream side of the leaflets and the edge 6 at the down-stream trailing side of the leaflets.

The edge 7 in Fig. 5 is a dull and straight edge, wherein the edge 6 is dull and serrated. The edge 7 in Fig. 6 is a dull and serrated edge with wave crests 21 and wave troughs 22 at the upstream edge 7 in open position of the heart valve 1. The edge 6 is dull and serrated.

In Fig. 7 the edges 6 and 7 have serrations 23, 24 in the form of waves and they are tapered in their extension from the main side 8 to the main side 9. In Fig. 8 the edge 6, herein also referred to as trailing edge, is serrated wherein the edge 7, herein also referred to as leading edge, has a straight and sharp form 25. In Fig. 8 the edge 6 is tapered in their extension from the main side 8 to the main side 9 wherein the edge 7 is tapered in the opposite direction.

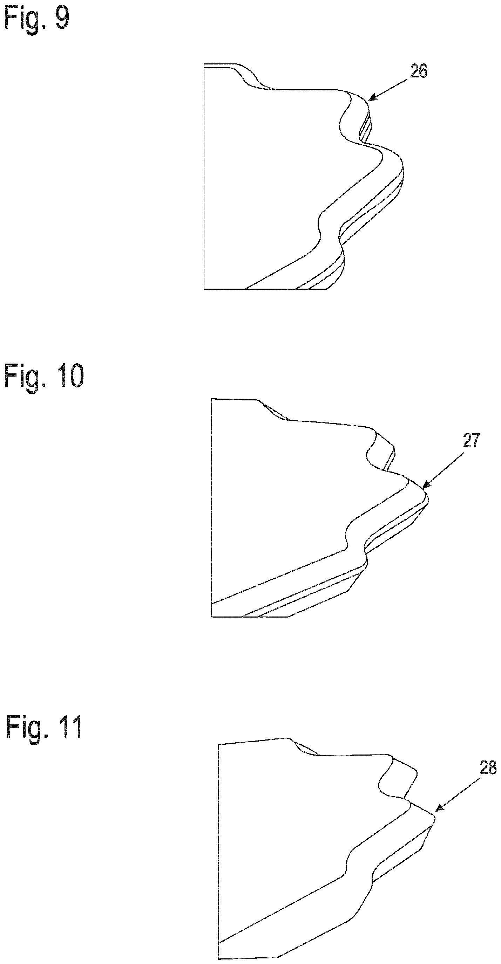

Preferably said tapered edge can protrude in a straight dull, a rounded or chamfered progress to one end section, wherein said end section more preferably protrudes over the whole length of said edge profile, as shown in Fig. 9-11.

The base body can be provided with striated profiles along the inner periphery as shown in Fig. 12-18.

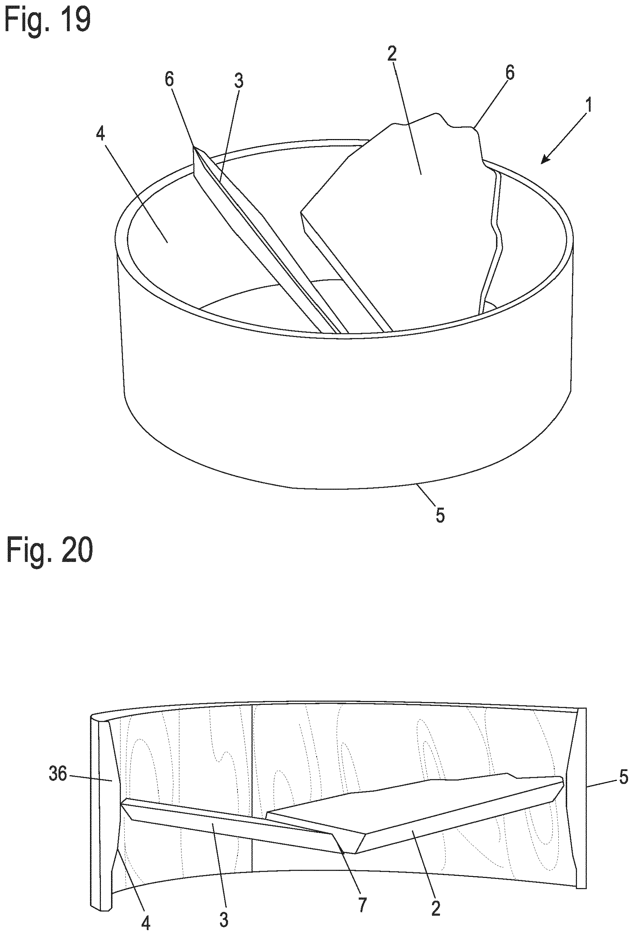

Fig. 19 and 20 disclose different states of a heart valve. Whereas Fig. 2 discloses a fully opening state, said Fig. 19 discloses a merely partly opening state and Fig. 20 a completely or nearly completely closed state of the heart valve.

The predescribed valves may be used equally in aortic, pulmonary, or mitral valve replacements

As demonstrated in figure 2 and 5-8 the serrated edge, herein also referred to as cutline, is defined by the superposition of sinusoidal waves of varying frequency and amplitude of the leaflet edge in the downstream side. The leading edge can be straight or serrated as well.

As mentioned before leading edge 7 can be either straight, fillet, or chamfer. The angle of the chamfer can be on the same side or on the opposite side. For both edges 6 and 7 the tapered, preferably fillet, also rounded end, or the chamfer end can have a flat mid-plane 26, 27 as shown in Fig. 9 and 10 or sharp edge 28 as shown in Fig. 5-8 and 11. For tri-leaflet mechanical heart valve and tissue heart valve, the serrated cutline applies to the edges of each one of the three leaflets, and adjacent edges have complementary curves.

As demonstrated in figure 12-18, the striated ring patterns are defined as the groove-like patterns on the inner wall of the ring, containing peaks, herein also referred to as ribs 30, 32, and valleys herein also referred to as grooves 31, 33. Said pattern of ribs and waves 30, 31, 32, 33 are matching the serrated trailing edge profile 6 of the leaflets 2, 3. These grooves can be aligned with the flow direction or tilted with an angle to the mean flow direction to generate helicity, preferably swirling motion, in the flow of a blood stream. This can further help the blood flow to turn more easily along the aortic arch. The tilting angle can be arbitrary. The peaks and valleys also feature cross-sectional profiles that are thinner towards the upstream/downstream edges of the ring, but thicker at the middle section 36 of the annular base body 5. Alternatively, the thicker section 37 can be positioned towards one edge of said annular base body 5 compared to the other edge of said base body 5, especially at a distance to an edge with less than 25% of the length of the annular body 5. This ensures the opening and closing of the serrated leaflets 2, 3.

The inventive heart valves combine two flow modulation strategies into one: the serrated trailing edge to accelerate turbulence decay, and the striated ring to reduce the drag and add helicity to the flow. Both features are likely to help ameliorate the unphysiological flow in prosthetic heart valves, and consequently reduce the thrombo-genicity of the valves.

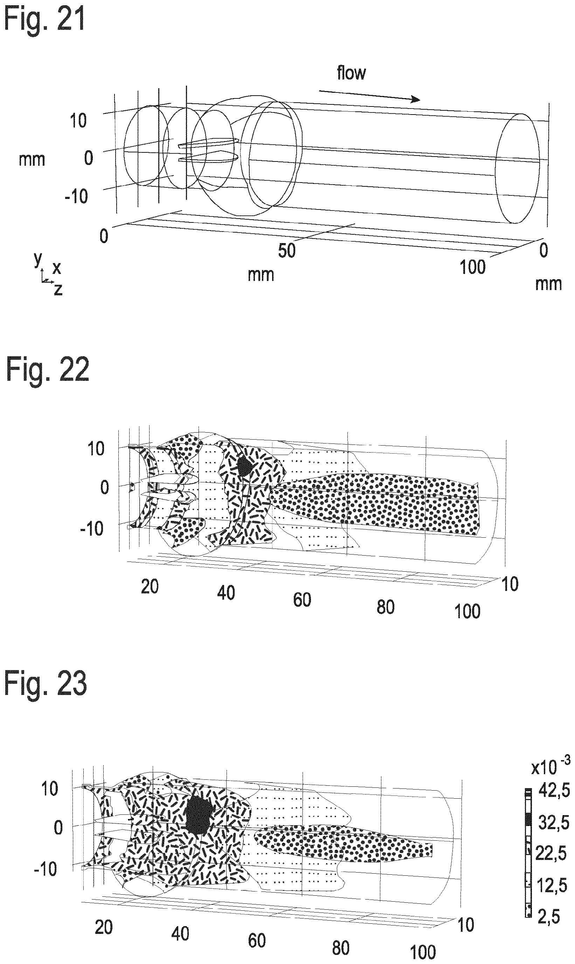

In Fig. 21 a model of an aorta with aortic sinuses and with a heart valve 1 in position is shown.

Isosurfaces of turbulent kinetic energy down-stream of the mechanical heart valve 101 of Fig. 1 is shown in Fig. 22.

Further Isosurfaces of turbulent kinetic energy down-stream of the inventive heart valve 1 of Fig. 2 Is shown in Fig. 23. The heart valve 1 of Fig. 2 is characterized by lower pressure gradient, lower turbulent kinetic energy, and lower shear stress level compared to the heart valve 101 of Fig. 1, which will be understood by comparing in Fig. 22 and 23. In Fig. 23, the flow profile is more concentrated towards the middle of the aorta. By using the inventive heart valve, the flow is guided closer along the main surfaces the leaflets and thus producing less turbulences and recirculation areas inside the flow.

Thus an inventive design for mechanical and biological prosthetic heart valves is provided. The design includes serrated leaflet edge designs of prosthetic heart valve leaflets that can be applied to new or existing mono-leaflet, bi-leaflet, and tri-leaflet mechanical or bioprosthetic heart valves. The design also includes new designs for a base body with striated profiles along the periphery on the inner surface of the ring that holds the leaflets. All design features may be used equally in aortic, pulmonary, or mitral valve replacements.

The design is flow optimized; thus, the risk of thrombosis is reduced by the design features of the leaflets of different heart valves shown in the figures.

**List of references**

| 1 | heart valve |
|---|---|
| 2 | leaflet |
| 3 | leaflet |
| 4 | inner wall surface |
| 5 | base body |
| 6 | edge profile / trailing edge |
| 7 | second edge / leading edge |
| 8 | main surface / main side |
| 9 | main surface / main side |
| 10 | wave crest |
| 11 | heart valve |
| 12 | prong |
| 13 | leaflet |
| 14 | leaflet |
| 15 | leaflet |
| 16 | edge profile |

| 17 | base body |
|---|---|
| 20 | wave trough |
| 21 | wave crest |
| 22 | wave trough |
| 23 | serration |
| 24 | serration |
| 25 | sharp form |
| 26 | mid-plane |
| 27 | mid-plane |
| 28 | sharp end |
| 30 | rib |
| 31 | groove |
| 32 | rib |
| 33 | groove |
| 36 | middle section |
| 37 | thicker section |
| 101 | heart valve |
| 111 | heart valve |
| L | longitudinal axis |
| A | pivot axis |
| B | pivot axis |
| D | diameter |

**Claims**

1. Heart valve (1, 11), especially prosthetic heart valve, comprising an annular base body (5, 17) and at least one leaflet (2, 3, 13, 14, 15) movably arranged relative to the base body (5, 17), wherein the heart valve (1, 11) has an opening state and a closed state, **characterized in that** the leaflet (2, 3, 13, 14, 15) has at least a serrated edge profile (6, 7, 16).

2. Heart valve according to claim 1, **characterized in that** the serrated edge profile (6, 7, 16) comprises a wave contour with a sequence of a plurality of wave crests (10, 21) and wave troughs (20, 22).

3. Heart valve according to claim 1 or 2, **characterized in that** the wave contour is formed by waves which are preferably running in a plate plane of the leaflet (2, 3) and which are more preferably formed as sinusoidal waves.

4. Heart valve according to one of the preceding claims, **characterized in that** the heart valve (1, 11) is designed as a surgical heart valve, either mechanical or biological.

5. Heart valve according to one of the preceding claims, **characterized in that** the leaflet (2, 3) is arranged pivotably about a pivot axis (A, B) and wherein the base body (5) has a wall with an inner wall surface (4) corresponding to the edge contour (6).

**6.** Heart valve according to one of the preceding claims, **characterized in that** all leaflets (2, 3, 13, 14, 15) of the heart valve (1, 11) have the serrated edge section (6, 7, 16), wherein the heart valve (1, 11) is preferably designed as a mono-leaflet, bi-leaflet or tri-leaflet heart valve.

**7.** Heart valve according to one of the preceding claims, **characterized in that** said serrated edge profile (6, 7, 16) is part of a downstream trailing side of the leaflet (2, 3, 13, 14, 15) of the heart valve (1, 11) in the opening state and wherein the serrated edge profile (6, 7, 16) is preferably part an arcuate shaped edge.

**8.** Heart valve according to one of the preceding claims, **characterized in that** the heart valve (1, 11) has a second edge (7) which is upstream of the leaflet of the heart valve (1, 11) in the opening state, wherein said second edge (7) has a serrated, in particular wavy, and/or chamfered edge profile.

**9.** Heart valve according to one of the preceding claims, **characterized in that** the edge profile (6) of either the first edge and/or the second edge (7) is at least partially straight or tapered, preferably tapered with a rounded or chamfered progress to one end section, wherein said end section more preferably protrudes over the whole length of said edge profile (6).

**10.** Heart valve according to one of the preceding claims, **characterized in that** the wall has ribs (30) that form a part of the inner wall surface (4), which preferably extend linearly and also preferably have an increasing thickness towards the center along the longitudinal axis (L) of the annular base body (5, 17).

**11.** Heart valve according to one of the preceding claims, **characterized in that** the wall has ribs (32) that form a part of the inner wall surface (4) and which extend in an arcuate or spiral shape, preferably at an angle ($\alpha$) of between 0° and 45°, more preferably 1° and 40°, to the longitudinal axis (L) of the annular base body (5, 17).

**12.** Heart valve according to one of the preceding claims, **characterized in that** the wall has at preferably arbitrary integer numbers larger than two of said ribs (30, 32), preferably at least eight of said ribs (30, 32), more preferably positioned equidistant from one another.

**13.** Heart valve according to one of the preceding claims, **characterized in that** the heart valve leaflets (13, 14, 15) are provided as part of a surgical tissue valve.

**14.** Heart valve according to one of the preceding claims, **characterized in that** the heart valve (11) is provided as a bioprosthetic heart valve, wherein the leaflet or leaflets (13, 14, 15) are formed from organic material, in particular organic or bio-engineered non-organic tissue material.

**15.** Heart valve according to one of the preceding claims, **characterized in that** the downstream trailing edges (6, 16) are preferably designed according to equation

$$\begin{cases} x_t = -r * cos(t) \\ y_t = -r * sin(t) + a * cos(b * t) \end{cases}$$

for $-\pi \le t \le 0$,

where $x_t$ and $y_t$ are spatial coordinates describing the shape of the edge, $r$ is the radius of the valve leaflet, $sin$ and $cos$ are sinusoidal functions, a is proportional to the amplitude of the wave, and b is proportional to the number of the crests of the wave.

# Fig. 1

# Fig. 2

# Fig. 3

# Fig. 4

Fig. 5

Fig. 6

Fig. 7

Fig. 8

Fig. 9

Fig. 10

Fig. 11

Fig. 12

30    31

5

4

Fig. 13

30    31

D

Fig. 14

36

L

36

**Fig. 15**

**Fig. 16**

**Fig. 17**

**Fig. 18**

## Fig. 19

## Fig. 20

Fig. 21

flow

Fig. 22

Fig. 23

x10$^{-3}$

42,5
32,5
22,5
12,5
2,5

Europäisches Patentamt

European Patent Office

Office européen des brevets

## EUROPEAN SEARCH REPORT

**Application Number**

EP 24 15 7743

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/346157 A1 (HUMAIR ARNAUD [CH]) 11 November 2021 (2021-11-11) | 1-9, 13-15 | INV. A61F2/24 |
| A | * paragraphs [0057] - [0075], [0086] - [0098]; figures 5-8,12,14-19 * | 10-12 | |
| | ----- | | |
| X | US 2021/059819 A1 (GUO WEI [CN] ET AL) 4 March 2021 (2021-03-04) | 1-9, 13-15 | |
| A | * paragraphs [0154] - [0205]; figures 1-11d * | 10-12 | |
| | ----- | | |

**TECHNICAL FIELDS SEARCHED (IPC)**

A61F

The present search report has been drawn up for all claims

| Place of search | Date of completion of the search | Examiner |
|---|---|---|
| The Hague | 5 July 2024 | Geuer, Melanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 15 7743

This annex lists the patent family members relating to the patent documents cited in the above-mentioned European search report.
The members are as contained in the European Patent Office EDP file on
The European Patent Office is in no way liable for these particulars which are merely given for the purpose of information.

05-07-2024

| Patent document cited in search report | Publication date | Patent family member(s) | | Publication date |
|---|---|---|---|---|
| US 2021346157 A1 | 11-11-2021 | CN | 111278387 A | 12-06-2020 |
| | | EP | 3697345 A1 | 26-08-2020 |
| | | JP | 7015918 B2 | 03-02-2022 |
| | | JP | 2021500155 A | 07-01-2021 |
| | | US | 2019117391 A1 | 25-04-2019 |
| | | US | 2021346157 A1 | 11-11-2021 |
| | | WO | 2019081453 A1 | 02-05-2019 |
| US 2021059819 A1 | 04-03-2021 | CN | 110353858 A | 22-10-2019 |
| | | EP | 3777767 A1 | 17-02-2021 |
| | | US | 2021059819 A1 | 04-03-2021 |
| | | WO | 2019196845 A1 | 17-10-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8834911 B
- US 9775708 B2
- US 10182907 B2
- US 20210212814 A1
- US 2021021815 A1
- US 20210212821 A1
- US 10478288 B2
- US 20040249452 A1